# FASCICULE DE BREVET EUROPEEN

(11) **EP 2 859 124 B1**
(45) Date de publication et mention de la délivrance du brevet: **25.04.2018**
(21) Numéro de dépôt: 13744827.0
(22) Date de dépôt: 12.06.2013
(51) Int. Cl.: C12Q 1/70, C12Q 1/68

(54) **METHODE DE QUANTIFICATION DES DIFFERENTES FORMES VIRALES DE L'ADN DU VIH**
VERFAHREN ZUR QUANTIFIZIERUNG VERSCHIEDENER VIRALER FORMEN DER DNS DES HIV-VIRUS
METHOD FOR QUANTIFYING DIFFERENT VIRAL FORMS OF THE DNA OF THE HIV VIRUS

(30) Priorité: 12.06.2012 FR 1255489
(43) Date de publication de la demande: 15.04.2015
(73) Titulaire: Centre National de la Recherche Scientifique (C.N.R.S.), 75016 Paris (FR); Ecole Normale Supérieure de Cachan, 94235 Cachan Cedex (FR); Université Paris Diderot - Paris 7, 75205 Paris Cedex 13 (FR)
(72) Inventeur: MUNIR, Soundasse, F-95300 Pontoise (FR); THIERRY, Sylvain, F-75018 Paris (FR); SUBRA, Frédéric, F-75009 Paris (FR); DEPREZ, Eric, F-77350 Boissettes (FR); DELELIS, Olivier, F-78220 Viroflay (FR)
(74) Mandataire: Regimbeau
(86) Numéro de dépôt international: PCT/IB2013/054809
(87) Numéro de publication internationale: WO 2013/186718

(56) Documents cités:
- WO-A1-00/14272
- WO-A2-2010/088491
- T. C. PIERSON ET AL: "Molecular Characterization of Preintegration Latency in Human Immunodeficiency Virus Type 1 Infection", JOURNAL OF VIROLOGY, vol. 76, no. 17, 1 septembre 2002 (2002-09-01), pages 8518-8531, XP055041411, ISSN: 0022-538X, DOI: 10.1128/JVI.76.17.8518-8513.2002 cité dans la demande
- YODER K E ET AL: "Real-time quantitative PCR and fast QPCR have similar sensitivity and accuracy with HIV cDNA late reverse transcripts and 2-LTR circles", JOURNAL OF VIROLOGICAL METHODS, ELSEVIER BV, NL, vol. 153, no. 2, 1 novembre 2008 (2008-11-01), pages 253-256, XP025495497, ISSN: 0166-0934, DOI: 10.1016/J.JVIROMET.2008.07.032 [extrait le 2008-09-17]
- BUTLER S L ET AL: "A quantitative assay for HIV DNA integration in vivo", NATURE MEDICINE, NATURE PUBLISHING GROUP, NEW YORK, NY, US, vol. 7, no. 5, 1 mai 2001 (2001-05-01), pages 631-634, XP001176851, ISSN: 1078-8956, DOI: 10.1038/87979
- SAKURAI YASUTERU ET AL: "DNA double strand break repair enzymes function at multiple steps in retroviral infection", RETROVIROLOGY, BIOMED CENTRAL LTD., LONDON, GB, vol. 6, no. 1, 15 décembre 2009 (2009-12-15), page 114, XP021068944, ISSN: 1742-4690
- Jean L. Mbisa ET AL: "Real-Time PCR Analysis of HIV-1 Replication Post-entry Events" In: "Clinical Applications of Mass Spectrometry", 1 janvier 2009 (2009-01-01), Humana Press, Totowa, NJ, XP055078719, ISSN: 1064-3745 ISBN: 978-1-60-761459-3 vol. 485, pages 55-72, DOI: 10.1007/978-1-59745-170-3_5, Paragraphe 3.7; tableaux 5.1, 5.2

## Description

### Domaine de l'invention

La présente invention concerne une méthode de quantification des différentes formes que peut adopter l'ADN viral du virus de l'immunodéficience humaine (VIH), notamment les formes linéaires, maturées ou non, et la forme circulaire 1-LTR.

### Arrière plan technique

Le virus de l'immunodéficience humaine (VIH) fait partie de la famille des *Retrovirus*. Lors de l'infection, l'ARN viral est converti en une molécule d'ADN viral linéaire double brin. L'ADN linéaire issu de la transcription inverse subit alors une étape de maturation à ses extrémités LTR (*Long Terminal Repeat*). Cette étape également dite de *3'-processing* consiste en une attaque endonucléolytique qui supprime deux bases, à chaque extrémité 3' du virus (Delelis et al. (2008) Retrovirology 5:114.). L'étape de maturation est indispensable pour rendre compatible les extrémités d'ADN avec la réaction d'intégration. Cette étape d'intégration du génome viral ADN est décrite dans la littérature comme étant une étape incontournable du cycle de réplication (Brown et al. (1990) Curr. Top. Microbiol. Immunol 157:19-48). En effet, l'intégration du virus, assurée par l'enzyme virale nommée intégrase, assure le maintien de l'information du virus au sein de la cellule mais aussi la production de nouvelles particules virales infectieuses et ainsi la propagation du virus. Il convient de noter que l'ADN viral peut aussi se retrouver sous formes circulaires (à 1 ou 2-LTR) dans la cellule mais que ces formes virales sont considérées comme n'ayant aucune fonction majeure dans le cycle de réplication et la progression virale (Sloan & Wainberg (2011) Retrovirology 8:52 et Wu (2008) Retrovirology 5:61). Elles sont qualifiées de molécules cul-de-sac ("*dead*-*end*").

Par conséquent, la quantification de l'ADN viral linéaire, seule forme virale compétente pour l'intégration du virus, s'avère un enjeu de premier plan. Dans ce cadre, plusieurs techniques basées sur la PCR (*Polymerase Chain Reaction*) existent pour quantifier les différentes formes d'ADN virales : forme intégrée, ADN viral circulaire à 2-LTR et ADN viral "total" (Graf et al. (2011) Plos Pathog. 7:e1001300). L'ADN viral total représente l'ensemble de tous les ADN viraux présents dans la cellule. Des méthodes de PCR ont été développées pour détecter l'ADN viral linéaire mais ne permettent pas de le quantifier (Pierson et al. (2002) Journal of Virology 17:8518-8531 ; Mohammed et al. (2011) Journal of Virology 85:4654-66). D'autre part, des méthodes de PCR ont été développées pour quantifier la forme circulaire 2-LTR de l'ADN viral (Butler et al. Nature Medicine, 7(5): 631-634, 2001; et WO 2010/088491). Par ailleurs, la technique du Southern blot permet de visualiser l'ensemble des formes d'ADN virales : circulaires ou linéaires (Zennou et al. (2000) Cell 101:173-85), mais cette technique n'est pas quantitative et est longue, peu sensible et nécessite du matériel radioactif.

Ainsi, à l'heure actuelle, aucune technique fiable et reproductible n'est disponible pour quantifier l'ADN viral linéaire et encore moins de quantifier l'ADN viral linéaire immature de l'ADN viral mature (c'est à dire ayant subi la réaction de *3'-processing*). Il faut préciser que la méthode par calcul (quantité d'ADN viral linéaire déduite par soustraction de la quantité d'ADN viral « total » de l'ADN viral 2-LTR, intégré et 1-LTR) n'est pas possible puisqu'une forme très représentée (les cercles à 1-LTR) n'est pas quantifiable à ce jour. De plus, ces techniques nécessitent de grande quantité de matériel viral.

### Description de l'invention

La présente invention découle de la mise au point, par les inventeurs, d'un procédé, basé sur la LM-PCR (*Linker-mediated PCR*), de quantification des formes linéaires et de la forme circulaire à 1-LTR du génome ADN du VIH-1.

La LM-PCR, bien connue de l'homme du métier, est notamment décrite par Mueller & Wold (1989) Science 246:780-786 et Pfeifer et al. (1989) Science 246:810-813.

Ainsi, la présente invention concerne un procédé de quantification des formes linéaires du génome ADN du virus de l'immunodéficience humaine 1 (VIH-1) dans un échantillon, dans lequel :
(i) on quantifie les formes linéaires totales du génome du VIH-1, et
(ii) on quantifie la forme linéaire non maturée du génome ADN du VIH-1
dans lequel on déduit la quantité de la forme linéaire maturée en 3' en soustrayant la quantité de la forme linéaire non maturée du génome ADN du VIH-1 quantifiée à l'étape (ii) de la quantité totale des formes linéaires du génome du VIH-1 quantifiée à l'étape (i) ;
dans lequel la quantification de l'étape (i) est réalisée par PCR quantitative à partir du produit de ligation obtenu en effectuant une réaction de ligation avec un oligonucléotide double brin qui permet une ligation avec la forme linéaire maturée en 3' et la forme linéaire non maturée en 3' du génome ADN du VIH-1 et comprend un bout collant de deux nucléotides de séquence 5'-GT-3' ; et
dans lequel l'étape (ii) est réalisée par PCR quantitative à partir du produit de ligation obtenu en effectuant une réaction de ligation avec un oligonucléotide double brin qui permet une ligation spécifiquement avec la forme linéaire non maturée en 3' du génome ADN du VIH-1 et comprend un bout franc

Le génome du VIH-1 peut être ARN, comme dans le virion. Il peut également être ADN, sous forme intégrée, dans le génome de l'hôte, ou sous forme non intégrée. Dans ce dernier cas, il peut alors se présenter sous forme linéaire ou sous forme circulaire. Les formes circulaires sont dénommées 1-LTR ou 2-LTR, selon qu'elles présentent un seul LTR (Long Terminal Repeat) ou deux. Les formes linéaires peuvent quant à elles être maturées ou non maturée à l'extrémité 3'.

La PCR (*Polymerase Chain Reaction,* réaction de polymérisation en chaine) est bien connue de l'homme du métier. Comme on l'entend ici, une PCR quantitative représente une réaction de polymérisation en chaine permettant de déterminer la quantité d'acide nucléique à amplifier initialement présent dans l'échantillon ayant subi la PCR. De nombreux types de PCR quantitative sont connus de l'homme du métier.

Comme cela apparaitra clairement à l'homme du métier, une « quantification » et une « quantité » selon l'invention sont absolues, et non relatives, c'est-à-dire qu'elles donnent accès à un nombre de molécules, à un nombre de moles, ou à une masse, ainsi qu'aux concentrations ou pourcentages correspondants.

Comme on l'entend ici, l'oligonucléotide double brin peut également être nommé oligonucléotide de liaison ou « *linker* », il est formé de l'association par hybridation de deux oligonucléotide dont au moins une partie de leurs séquences sont complémentaires.

La réaction de ligation est bien connue de l'homme du métier, elle consiste à créer une liaison covalente entre l'extrémité 3' d'un acide nucléique et l'extrémité 5' d'un autre acide nucléique. Elle est généralement mise en oeuvre à l'aide d'une ligase.

Selon l'invention, le linker défini ci-dessus permet une ligation avec la forme linéaire maturée en 3' et la forme linéaire non maturée en 3' du génome ADN du VIH-1 et comprend un bout collant de deux nucléotides de séquence 5'-GT-3', et on déduit de la PCR quantitative la quantité totale des formes linéaires du génome du VIH-1 dans l'échantillon. Plus préférablement, l'oligonucléotide de liaison est alors formé de l'association d'une séquence 5'-GCGGTGACCCGGGAGATCTGAATTC-3' (SEQ ID NO : 5), ou une séquence présentant au moins 90% d'identité avec SEQ ID NO : 5, et d'une séquence 5'-GTGAATTCAGATC-3 (SEQ ID NO : 6), ou une séquence ayant au moins 90% d'identité avec SEQ ID NO : 6.

Comme on l'entend ici, un acide nucléique double brin est dit présenter un bout collant lorsque au moins l'une des extrémités de l'acide nucléique possède un brin protubérant qui ne peut pas s'apparier pas à l'autre brin. A *contrario*, un acide nucléique double brin est dit présenter un bout franc lorsqu'à au moins l'une des extrémités de l'acide nucléique les deux brins sont complètement appariés.

Une séquence présentant au moins 90% d'identité avec SEQ ID NO: X selon l'invention, présente de préférence au moins 95% d'identité avec SEQ ID NO : X. Cette séquence diffère notamment de SEQ ID NO: X par l'insertion, la suppression ou la substitution d'au moins un nucléotide. Comme on l'entend ici, le pourcentage d'identité entre deux séquences est défini comme le nombre de positions pour lesquelles les bases sont identiques lorsque les séquences sont alignées de manière optimale, divisé par le nombre total de bases de la plus grande des deux séquences. Deux séquences sont dites alignées de manière optimale lorsque le pourcentage d'identité est maximal. Par ailleurs, comme cela apparaitra de manière claire à l'homme du métier, il peut être nécessaire de faire appel à des ajouts de positions lacunaires (des « *gaps* ») de manière à obtenir un alignement optimal entre les deux séquences. En outre, la séquence présentant au moins 90% d'identité avec SEQ ID NO : X selon l'invention conserve la fonction, notamment d'amorce ou de sonde, de SEQ ID NO : X, c'est-à-dire qu'elle permet une amplification ou une détection de l'acide nucléique à amplifier.

Selon l'invention, l'oligonucléotide de liaison défini ci-dessus permet une ligation spécifiquement avec la forme linéaire non maturée en 3' de la forme linéaire du génome du VIH-1 et comprend un bout franc, et on déduit de la PCR quantitative la quantité de la forme linéaire non maturée du génome du VIH-1 dans l'échantillon. Plus préférablement, l'oligonucléotide de liaison est alors formé de l'association d'une séquence 5'-GCGGTGACCCGGGAGATCTGAATTC-3' (SEQ ID NO : 5), ou une séquence ayant au moins 90% d'identité avec SEQ ID NO : 5, et d'une séquence 5'-GAATTCAGATC-3' (SEQ ID NO : 7), ou une séquence ayant au moins 90% d'identité avec SEQ ID NO : 7.

Selon l'invention, la quantité de la forme linéaire maturée du génome du VIH-1 dans l'échantillon est déterminée par soustraction de (i) la quantité de la forme linéaire non maturée du génome du VIH-1 dans l'échantillon déterminée selon l'invention à (ii) la quantité totale des formes linéaires du génome du VIH-1 dans l'échantillon déterminée selon l'invention.

De préférence également, la PCR quantitative selon l'invention est une PCR en temps réel, plus préférablement conduite avec des sondes du type sonde d'hybridation.

La PCR en temps réel est bien connue de l'homme du métier. Brièvement, la PCR en temps réel associe l'amplification d'un acide nucléique et la détection par fluorescence de l'acide nucléique amplifié. On effectue généralement une PCR classique en présence de sondes, notamment de sondes d'hybridation, c'est-à-dire des sondes qui s'hybrident à l'acide nucléique amplifié et qui émettent un signal fluorescent en réponse à cette hybridation. L'émission de fluorescence est mesurée en fonction des cycles de PCR. Le cycle de PCR pour lequel le signal de florescence émis est mesuré au dessus d'un niveau seuil, généralement au dessus du niveau basal ou du niveau de fond de fluorescence, est nommé le cycle seuil (Ct). Il a été montré que Ct est proportionnel au logarithme décimal de la quantité d'acide nucléique à amplifier initialement présent dans la réaction de PCR (voir par exemple "Real-time PCR" in Advanced Methods S., Dorak M T. ed, Taylor and Francis, Oxford, 2006).

Parmi les nombreuses sondes d'hybridation selon l'invention connue de l'homme du métier applicables selon l'invention, il est notamment possible de citer les sondes du type *Molecular Beacon* qui sont par exemple décrite dans la demande internationale WO 98/10096.

De manière préférée, la PCR quantitative selon l'invention est réalisée en deux étapes : une première étape d'amplification sans quantification de l'acide nucléique amplifié et une deuxième étape d'amplification avec quantification de l'acide nucléique amplifié.

De manière davantage préférée, la première étape de la PCR quantitative selon l'invention est réalisée avec une amorce comprenant une séquence 5'-GCGGTGACCCGGGAGATCTGAATTC-3' (SEQ ID NO: 5), telle qu'une séquence 5'-GCGCGCGGCGGTGACCCGGGAGATCTGAATTC-3' (SEQ ID NO: 25), ou une séquence présentant au moins 90% d'identité avec SEQ ID NO : 5 ou 25, et avec une amorce comprenant une séquence 5'-CTCGCCTCTTGCCGTGCGCG-3' (SEQ ID NO : 9), ou une séquence présentant au moins 90% d'identité avec SEQ ID NO : 9.

De manière davantage préférée également la deuxième étape de la PCR quantitative selon l'invention est réalisée avec une amorce comprenant une séquence 5'-GCGGTGACCCGGGAGATCTGAATTC-3' (SEQ ID NO : 5), ou une séquence présentant au moins 90% d'identité avec SEQ ID NO : 5, et une amorce comprenant une séquence 5'-GAGTCCTGCGTCGAGAGATC-3' (SEQ ID NO: 26), ou une séquence présentant au moins 90% d'identité avec SEQ ID NO : 26, et une sonde d'hybridation comprenant une séquence 5'-CCCTCAGACCCTTTTAGTCAGTGTGGAA-3' (SEQ ID NO: 27), ou une séquence présentant au moins 90% d'identité avec SEQ ID NO: 27, et/ou une sonde d'hybridation comprenant une séquence 5'-TCTCTAGCAGTGGCGCCCGAACAG-3' (SEQ ID NO : 28), ou une sonde présentant au moins 90% d'identité avec SEQ ID NO : 28.

Par ailleurs, dans le procédé défini ci-dessus, on préfère que la première étape de la PCR quantitative comprenne de 9 à 30 cycles, plus préférablement 10 à 14 cycles, et encore plus préférablement environ 12 cycles.

L'étape de déduction de la quantité de la forme linéaire maturée en 3' et/ou de la forme linéaire non maturée en 3' du génome ADN du VIH-1 dans l'échantillon peut notamment être mise en oeuvre par référence à une courbe de calibration. Cette courbe de calibration peut notamment être obtenue en réalisant les deux premières étapes du procédé de quantification selon l'invention à partir de quantités connues de molécules d'ADN double brin mimant la forme linéaire maturée en 3' et/ou la forme linéaire non maturée en 3' du génome ADN du VIH-1. Ces quantités connues de molécules d'ADN mimant la forme linéaire maturée en 3' et/ou la forme linéaire non maturée en 3' du génome ADN du VIH-1 sont de préférence obtenues par digestion de quantités connues d'un plasmide à l'aide d'enzymes de restriction permettant notamment de libérer des molécules d'ADN double brin ayant au moins un bout franc, pour les molécules mimant la forme linéaire non maturée, ou au moins un bout collant de 2 nucléotides, pour les molécules mimant la forme linéaire non maturée. Par ailleurs, on préfère que les molécules d'ADN double brin mimant la forme linéaire maturée en 3' et/ou la forme linéaire non maturée en 3' du génome ADN du VIH-1 aient sensiblement la même taille et/ou sensiblement la même séquence que ces dernières. Toutefois, des variations de tailles ou de séquences limitées sont acceptables. En particulier, le bout collant de 2 nucléotides de la molécule d'ADN double brin mimant la forme linéaire maturée du génome ADN du VIH-1 peut avoir une séquence différente de celui de la forme linéaire maturée ; dans ce cas, l'homme du métier comprendra qu'il convient de modifier en conséquence le bout collant de l'oligonucléotide de liaison adapté pour une ligation avec cet ADN double brin et utilisé dans la préparation de la courbe de calibration, pour qu'il soit complémentaire de celui de l'ADN double brin.

Par ailleurs, l'invention concerne également un procédé de quantification de la forme circulaire à 1-LTR du génome ADN du VIH-1 dans un échantillon, dans lequel on effectue une PCR quantitative selon l'invention à l'aide de deux amorces comprenant respectivement une séquence 5'-GCGCTTCAGCAAGCCGAGTCCT-3' (SEQ ID NO : 1), ou une séquence présentant au moins 90% d'identité avec SEQ ID NO: 1, et une séquence 5'-GTCACACCTCAGGTACCTTTAAGACCAATGAC-3' (SEQ ID NO : 2), ou une séquence présentant au moins 90% d'identité avec SEQ ID NO : 2.

De préférence, la PCR quantitative est conduite avec une sonde d'hybridation comprenant la séquence 5'-CACAACAGACGGGCACACACTACTTGA-3' (SEQ ID NO : 3), ou une séquence présentant au moins 90% d'identité avec SEQ ID NO : 3 et/ou avec une sonde d'hybridation comprenant la séquence 5'-CACTCAAGGCAAGCTTTATTGAGGC-3' (SEQ ID NO : 4), ou une séquence présentant au moins 90% d'identité avec SEQ ID NO : 4.

De préférence également, la PCR quantitative mise en oeuvre dans le procédé de quantification de la forme circulaire à 1-LTR du génome ADN du VIH-1 selon l'invention requiert la définition d'une durée précise de l'étape d'élongation de la PCR. Ainsi, on préfère que l'étape d'élongation, c'est-à-dire l'étape conduite à une température favorisant l'activité de l'ADN polymérase thermostable, ait une durée de 20 à 30 secondes, plus préférablement de 23 à 27 secondes, et encore plus préférablement d'environ 25 secondes.

L'invention concerne également un procédé de criblage de composés susceptibles de modifier la quantité d'une forme non intégrée du génome du VIH-1, comprenant la mise en oeuvre d'un procédé de quantification des formes linéaires ou de la forme circulaire à 1-LTR tel que défini ci-dessus. Dans un mode de réalisation préféré du procédé de criblage selon l'invention, les composés sont susceptibles d'inhiber la maturation de la forme linéaire du génome ADN du VIH-1 et on met en oeuvre un procédé de quantification des formes linéaires telles que définies ci-dessus.

L'invention sera davantage explicitée à l'aide des Exemples et des Figures qui suivent.

### Description des figures

Figures 1A, 1B et 1C

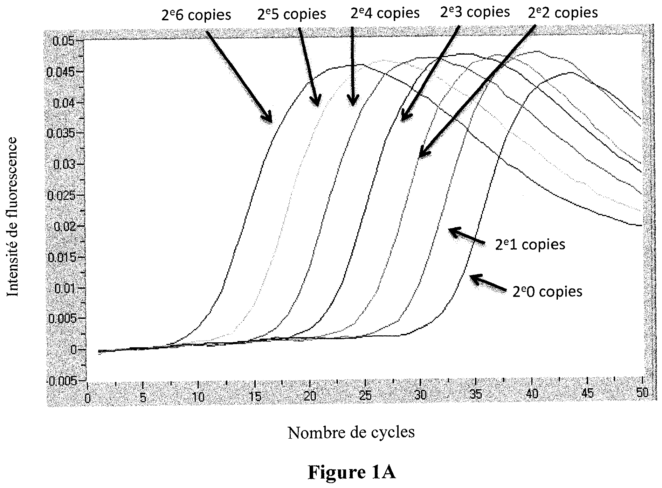

   Figure 1A : PCR sur dilutions du plasmide pLIN-HIV-ScaI. Des dilutions successives de plasmide pLIN-HIV-ScaI ont été effectuées (de 2^{e}6 copies à 2^{e}0 copies). L'efficacité calculée de la PCR à l'aide de la méthode de la dérivée seconde fournie par le logiciel de quantification du *Light Cycler,* version 3.5 (Roche Diagnostics) est de 90%. L'intensité de Fluorescence (axe des ordonnées) est suivie en fonction du nombre de cycles (axe des abscisses).

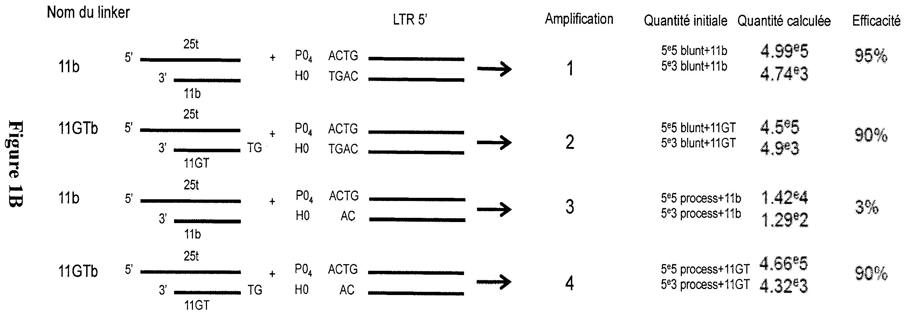

   Figure 1B : Résultats des différentes PCR utilisant les linkers et les différents substrats étudiés.

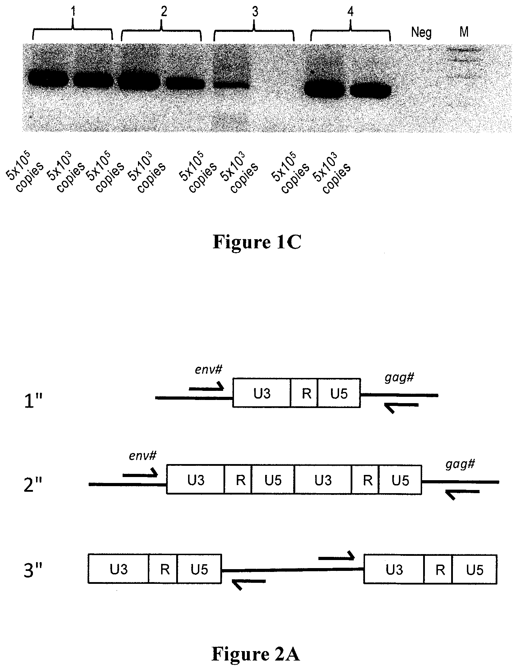

   Figure 1C : Photographie d'un gel d'électrophorèse dans lequel les produits d'amplification ont été mis à migrer.
Figures 2A, 2B, 2C et 2D
   Figure 2A : Schéma de l'amplification utilisant les amorces pour la quantification des cercles 1-LTR sur les substrats 1-LTR (1), 2-LTR (2) et sur l'ADN viral linéaire (3).

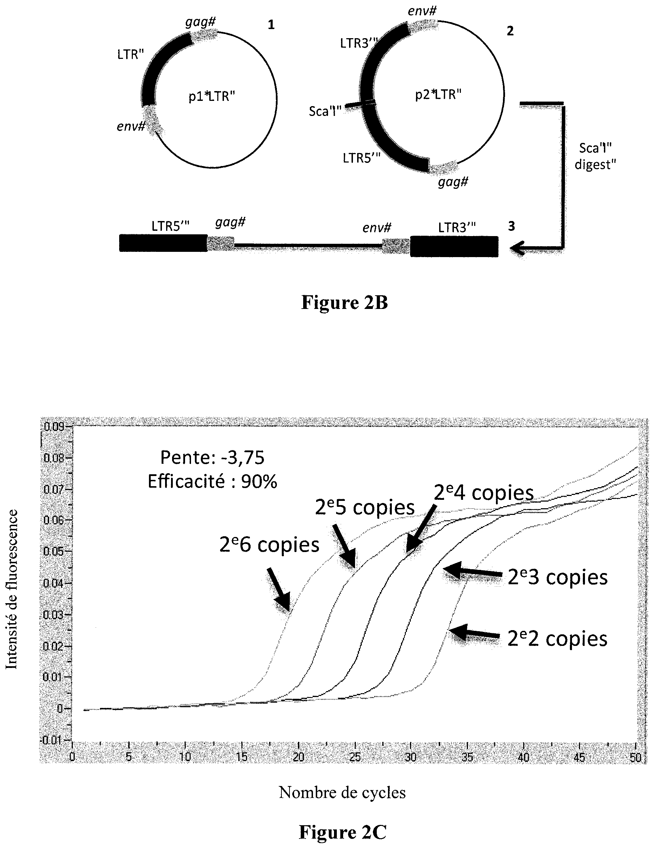

   Figure 2B : Plasmides utilisés : Plasmide 1-LTR (1), 2-LTR (2) et ADN mimant l'ADN viral linéaire après digestion du plasmide 2-LTR par l'enzyme ScaI (3).
   Figure 2C : Efficacité de la PCR quantitative 1-LTR. Des dilutions successives du plasmide 1-LTR (2^{e}6 copies à 2^{e}2 copies) ont été effectuées. L'intensité de Fluorescence (axe des ordonnées) est suivie en fonction du nombre de cycles (axe des abscisses).

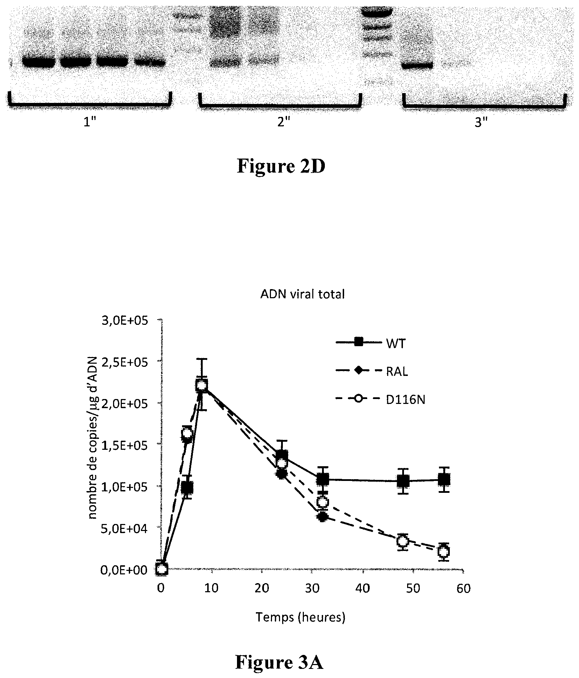

   Figure 2D : Photographie d'un gel d'électrophorèse dans lequel les produits d'amplification obtenus à partir du plasmide 1-LTR (1), du plasmide 2-LTR (2) et de l'ADN mimant l'ADN viral linéaire après digestion du plasmide 2-LTR par l'enzyme ScaI (3) ont été mis à migrer.
Figures 3A, 3B, 3C, 3D, 3E et 3F
   Cinétiques des différentes formes d'ADN viral après infection de cellules MT4 par le virus WT (en présence ou non de RAL) ou par un virus D116N. La condition WT sans Raltégravir est symbolisée par des carrés noirs. La condition WT avec Raltégravir est symbolisée par des diamants noirs. La condition D116N est symbolisée par des ronds blancs. Le nombre de copies d'ADN viral, normalisé par la quantité d'ADN cellulaire total (axe des ordonnées), est donné en fonction du temps en heures (axe des abscisses).
   Figure 3A : cinétique de l'ADN viral total.

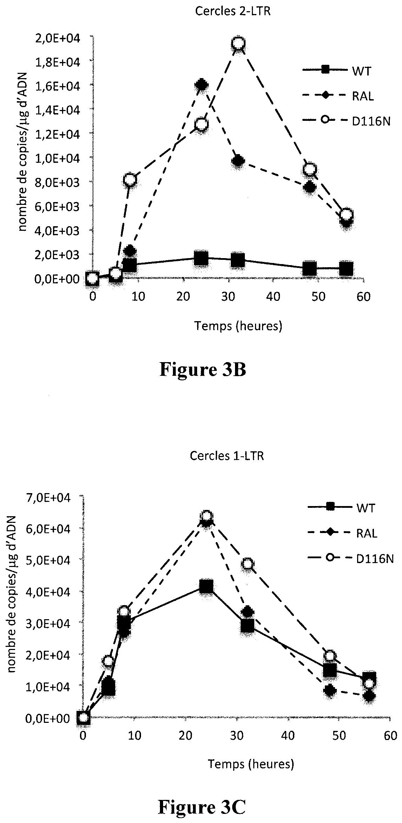

   Figure 3B : cinétique des cercles 2-LTR.
   Figure 3C : cinétique des cercles 1-LTR.

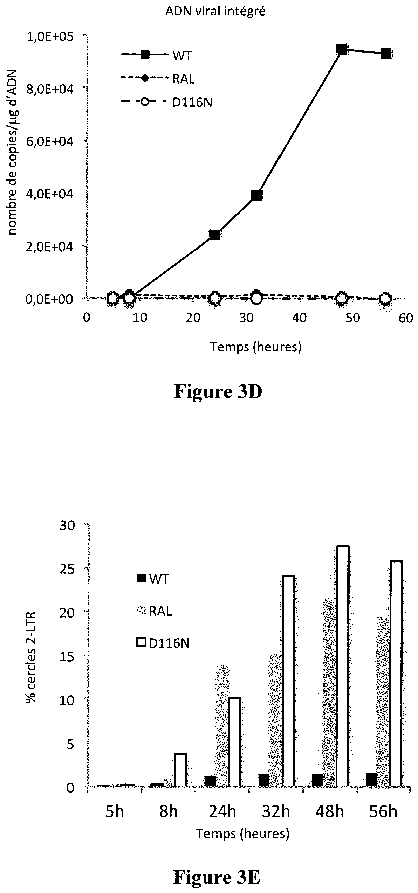

   Figure 3D : cinétique de l'ADN viral intégré.
   Figure 3E : pourcentage des cercles 2-LTR (quantité de cercles 2-LTR sur l'ADN viral total, axe des ordonnées) en fonction du temps (axe des abscisses, en heures) pour la condition WT sans Raltégravir (WT, barres noires), la condition WT avec Raltégravir (RAL, barres grises) et la condition D116N (barre blanches).

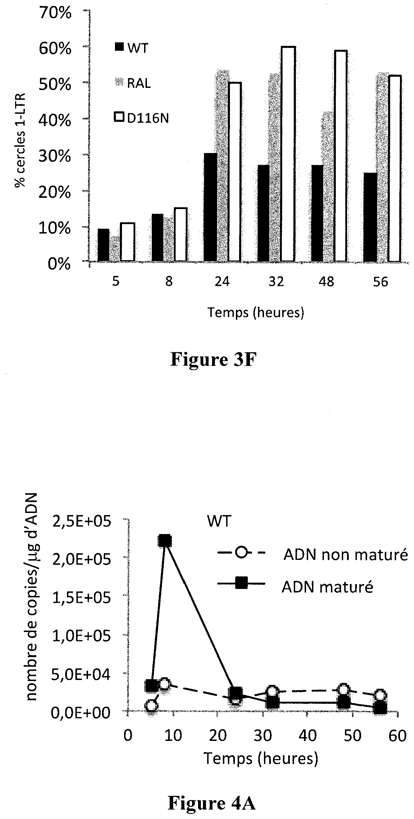

   Figure 3F : ainsi que le pourcentage des cercles 1-LTR (quantité de cercles 1-LTR sur l'ADN viral total axe des ordonnées) en fonction du temps (axe des abscisses, en heures) pour la condition WT sans Raltégravir (WT, barres noires), la condition WT avec Raltégravir (RAL, barres grises) et la condition D116N (barre blanches).
Figures 4A, 4B, 4C et 4D

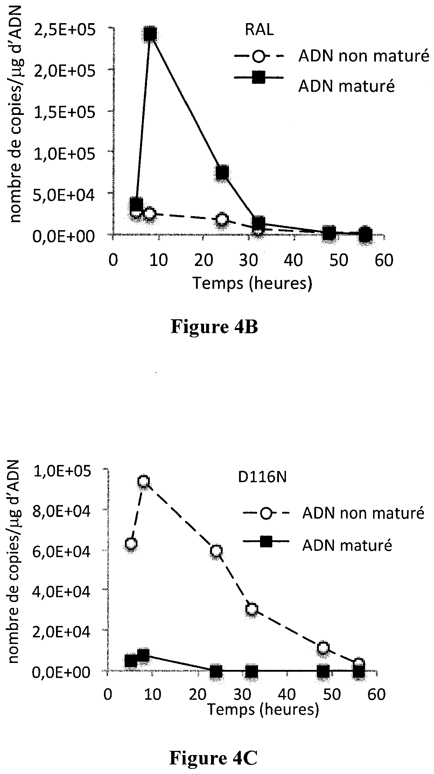

   Figures 4A, 4B et 4C : Cinétique de l'ADN viral linéaire maturé et non maturé après infection de cellules CEM par le virus WT (en présence ou non de Raltégravir, respectivement Figure 4B et Figure 4A) ou par le virus D116N (Figure 4C). La quantité d'ADN non maturé est symbolisée par un rond blanc. La quantité d'ADN maturé est symbolisée par un carré noir. Le nombre de copies d'ADN viral, normalisé par la quantité d'ADN cellulaire total (axe des ordonnées), est donné en fonction du temps en heures (axe des abscisses).

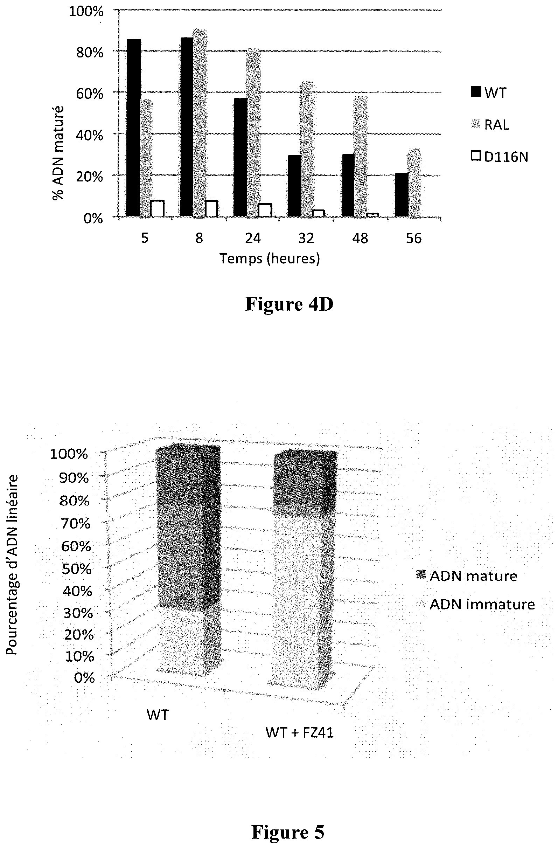

   Figure 4D : Le pourcentage d'ADN maturé (quantité d'ADN maturé sur la quantité d'ADN linéaire total) est représenté pour les trois conditions : WT (noir), WT en présence de Raltégravir (gris) et D116N (en blanc).
Figure 5
   Les cellules CEM ont été infectées par le virus pNL4.3 en absence (WT) ou en présence de 20µM de FZ41 (WT+FZ41). l'ADN viral maturé et non maturé est quantifié 24 heures après infection. La proportion de chaque espèce d'ADN viral linéaire maturé (gris) et non maturé (blanc) est représentée.

### EXEMPLES

### Exemple 1

### Matériels et méthodes

*Cellules et virus.* La lignée cellulaire lymphoïde CEM (NIH, référence catalogue 117) a été cultivée en milieu RPMI1640 (Invitrogen, Cat#61870-044) supplémenté par 10% de sérum de veau foetal (SVF) (PAA, Lot#A10109-113). Les cellules 293T et HeLa ont été cultivées en DMEM (Invitrogen, Cat#31966-047) (*Dulbecco's modified Eagle medium*) supplémenté par 10% de SVF. Les stocks de VIH-1 ont été préparés par transfection de cellules 293T avec les différents clones moléculaires dérivés de pNL4-3 (Gelderblom et al. (2008) Retrovirology 9;5:60). Les virus Δenv NLENG1-ES-IRES WT et NLENG1-ES-IRES D116N (Gelderblom et al. (2008) Retrovirology 9;5:60) codent respectivement le type sauvage et le mutant D116N catalytiquement inactif de l'intégrase. L'élément IRES est nécessaire pour l'expression de la GFP. Le pseudotypage des virus Δenv a été effectué par co-transfection de cellules 293T avec un plasmide VSV-G (Addgene, plasmide 8454) à l'aide de la méthode au phosphate de calcium. Les surnageants viraux ont été filtrés à travers un filtre à diamètre de pores de 0,45 µm (Sartorius, référence 16537K) puis congelés à - 80°C.

*Infection virale.* Le contenu en antigène p24^{gag} du VIH-1 des surnageants de culture a été déterminé par ELISA (Perkin-Elmer Life Sciences, Paris, France référence : NEK050B001KT). 40ng d'antigène p24^{gag} pour 10⁶ cellules, ce qui correspond à une multiplicité d'infection (m.o.i.) de 0.1, ont été utilisés pour l'infection. Lorsque nécessaire, les cellules ont été traitées en présence de l'inhibiteur d'intégrase raltégravir (RAL) à 500nM. 2 à 5 millions de cellules ont été collectées à différents temps après infection. Les cellules ont ensuite été lavées en PBS, puis centrifugées, et les culots cellulaires secs ont été congelés à -80°C jusqu'à leur utilisation. L'ADN cellulaire total des cellules infectées de la lignée cellulaire CEM a été purifié à l'aide du kit *QIAamp DNA Blood mini kit* (Qiagen, référence 51104) selon les instructions du fabriquant. Afin de dégrader les plasmides de transfection résiduels, l'ADN extrait a été incubé avec 5U de *Dpn*I (NEB, référence R0176S) dans un tampon comprenant 20mM Tris pH 7.9 pendant 4 heures à 37°C.

*Plasmides.* Quatre plasmides ont été construits : p1-LTR, p2-LTR, pLIN-HIV-ScaI et pLIN-HIV-NdeI.

pLIN-HIV-ScaI a été construit comme suit : amplification par PCR à l'aide de l'oligonucléotide 25tSca-HIV (5'-GCGGTGACCCGGGAGATCTGAATTCAGTACTGGAAGGGCTAATTTGGTCCC-3', SEQ ID NO : 8) et de l'oligonucléotide MS1 (5'-CTCGCCTCTTGCCGTGCGCG-3', SEQ ID NO : 9) en utilisant le plasmide pNL4.3 comme matrice. Le produit d'amplification a alors été purifié et introduit dans le vecteur pGEMT-easy (Proméga, référence A1360). Les nucléotides soulignés représentent la séquence d'ADN virale du LTR5' (région U3). Il est à préciser qu'un site *Sca*I (5'-AGTACT-3', SEQ ID NO : 10) existe en position 315 dans le plasmide pNL4.3. Ce site *Sca*I a été modifié en un site ne permettant pas la coupure par l'enzyme de restriction *Sca*I (5'AGTACT-3' muté en 5'-AGAACT-3', SEQ ID NO : 11) par un procédé de mutagénèse dirigée site-spécifique (*QuikChange Lightning Kits,* Agilent) à l'aide des oligonucléotides de séquences 5'-CCCGAGAGCTGCATCCGGAGAACTACAAAGACTGCTGACATCG-3' (SEQ ID NO: 12) et 5'-CGATGTCAGCAGTCTTTGTAGTTCTCCGGATGCAGCTCTCGGG-3', SEQ ID NO : 13). La digestion par *Sca*I et *Aat*II conduit à un fragment mimant l'extrémité non-maturée de l'ADN viral. Après purification, ce fragment est utilisé pour mettre en oeuvre une réaction de ligation avec les oligonucléotides de liaison double brin (ou « linker ») 25t/11b ou 25t/11GT.

pLIN-HIV-NdeI a été construit par mutagénèse dirigée site-spécifique (*QuikChange Lightning Kits*, Agilent) du plasmide pLIN-HIV-ScaI, en mutant le site *Sca*I (5'-AGTACT-3', SEQ ID NO : 10) en un site *Nde*I (5'-CATATG-3', SEQ ID NO : 14) à l'extrémité de l'ADN viral à l'aide des oligonucléotides de séquences 5'-CCGGGAGATCTGAATTCAGTCATATGGAAGGGCTAATTTGGTCC-3' (SEQ ID NO : 15) et 5'-GGACCAAATTAGCCCTTCCATATGACTGAATTCAGATCTCCCGG-3' (SEQ ID NO : 16). La digestion par *Nde*I et *Aat*II (dont le site de digestion n'est présent que dans le vecteur pGEMT-easy) conduit à un fragment mimant l'extrémité 3' maturée de l'ADN viral. Après purification, ce fragment est utilisé comme standard de la PCR visant à quantifier l'ADN maturé en 3'. Il est important de noter que la double digestion *Nde*I et *Aat*II permet d'obtenir un fragment mimant l'ADN maturé du VIH-1 (extrémité cohésive de 2 paires de bases 5'-TA-3'). Cependant, la nature de ces bases diffère avec celles trouvées à l'extrémité du génome maturé (5'-AC-3'). Toutefois, la nature de ces bases n'interfère en rien avec la méthode de quantification de l'ADN viral maturé.

Les amorces 1LTR LA1 (5'-GCGCTTCAGCAAGCCGAGTCCT-3', SEQ ID NO : 1) et 1LTR LA16 (5'-GTCACACCTCAGGTACCTTTAAGACCAATGAC-3', SEQ ID NO : 2) ont été utilisées pour amplifier par PCR la région env-LTR-gag contenue dans les molécules circulaires à 1-LTR à partir d'extraits de cellules infectées par le VIH-1. Le plasmide p1-LTR a alors été obtenu en clonant le produit d'amplification dans le vecteur pGEMT-easy.

Le plasmide p2-LTR a été construit comme suit : les amorces ScaI-LTR3' (5'-CGGGAGATCTGAATTCAGTACTGCTAGAGATTTTCCACACTGACTAAAAG-3', SEQ ID NO: 17) et 1LTR LA16 (5'-GTCACACCTCAGGTACCTTTAAGACCAATGAC-3', SEQ ID NO: 2) ont été utilisées pour amplifier la région *env*-LTR3' et le produit d'amplification a été inséré dans le vecteur pGEMT-easy pour donner le plasmide intermédiaire pSca-LTR3'. Le site *Sca*I (5'-AGTACT-3', SEQ ID NO : 10), présent dans le LTR3' en position 10715 du génome viral a été muté en 5'-AGCACT-3' (SEQ ID NO : 18) par un procédé de mutagénèse dirigée site-spécifique (*QuikChange Lightning Kits,* Agilent) à l'aide des oligonucléotides de séquences 5'-AGCTGCATCCGGAGCACTTCAAGAACTGCT-3' (SEQ ID NO: 19) et 5'-AGCAGTTCTTGAAGTACTCCGGATGCAGCT-3' (SEQ ID NO : 20). Le plasmide pSca-LTR3' et le plasmide pLIN-HIV-ScaI ont été digérés par l'enzyme *Sca*I. Les fragments obtenus, respectivement de taille 2520pb et 1920pb ont été purifiés et ligaturés pour obtenir le plasmide p2-LTR. Enfin le site *Sca*I (5'-AGTACT-3', SEQ ID NO : 10) présent dans le vecteur pGEMT-easy (en position 1890 du vecteur) a été muté en un site 5'-AATACT-3' (SEQ ID NO: 21) à l'aide des oligonucléotides 5'-GTGACTGGTGAATACTCAACCAAGTCATTCTGAG-3' (SEQ ID NO: 22) et 5'-CTCAGAATGACTTGGTTGAGTATTCACCAGTCAC-3' (SEQ ID NO: 23), afin de permettre la linéarisation du plasmide p2-LTR par digestion de celui-ci par l'enzyme *Sca*I.

*Quantification des formes linéaires non maturées et maturées en 3' de l'ADN du VIH-1 par LM-PCR*. La stratégie utilisée pour quantifier l'ADN viral linéaire est basée sur la ligation d'un oligonucléotide de liaison (ou « linker ») aux terminaisons des extrémités virales suivie de deux PCR selon le protocole de Pierson *et al.* (Pierson et al. (2002) Journal of Virology 17:8518-8531). Des améliorations majeures ont été apportées afin de mettre en oeuvre une approche de type PCR quantitative.

Le linker constitué des deux oligonucléotides 25t et 11b (25t: 5'-GCGGTGACC CGGGAGATCTGAATTC-3', SEQ ID NO : 5, 11b: 5'-GAATTCAGATC-3', SEQ ID NO : 7) (linker 11b) et le linker constitué des oligonucléotides 25t et 11GT (25t: 5'-GCGGTGACCCGGGAGATCTGAATTC-3', SEQ ID NO: 5, 11GT: 5'-GTGAATTCAGATC-3', SEQ ID NO: 6) (linker 11GTb), ont été respectivement utilisés pour la quantification de l'ADN linéaire non maturé du VIH-1 et de l'ADN linéaire maturé du VIH-1.

Pour la quantification de l'ADN viral linéaire non mature, l'efficacité de la procédure de ligation-amplification a été déterminée par l'ajout de l'oligonucléotide de liaison (linker 11b) à des dilutions en série du fragment de digestion de pLIN-HIV-ScaI obtenu par digestion par les enzymes *Sca*I et *Aat*II. Les dilutions en série de ce fragment constituent la gamme de calibration de l'ADN non maturé.

Pour la quantification de l'ADN viral linéaire maturé, l'efficacité de la procédure de ligation-amplification a été déterminée par ajout de l'oligonucléotide de liaison (linker 11TAb) constitué des oligonucléotides 25t et 11TA (11TA: 5'-TAGAATTCAGATC-3', SEQ ID NO : 24) à des dilutions en série de fragments obtenus par digestion de pLIN-HIV-NdeI par *Nde*I et *Aat*II. Les dilutions en série de ce fragment constituent la gamme de calibration de l'ADN maturé.

Il est à noter que lors de l'infection de cellules par le virus VIH-1, l'extrémité de l'ADN viral linéaire maturé diffère de l'extrémité de la gamme de calibration, c'est pour cela que la quantification de l'ADN viral maturé au sein des cellules infectées est effectuée à l'aide du linker 11GTb au lieu du linker 11TAb. Cette différence n'influe en rien sur la quantification de l'ADN viral maturé. La quantification de l'ADN viral linéaire maturé est donc obtenue grâce par comparaison à la gamme de calibration.

Les différentes courbes de calibration ont été réalisées en présence d'ADN de cellules non infectées afin de vérifier que l'efficacité de la ligation/amplification n'était pas influencée par la captation d'oligonucléotide de liaison par l'ADN des cellules non infectées.

Les oligonucléotides de liaison ont été associés en chauffant chaque brin complémentaire (à la concentration finale de 3µM) à 95°C pendant 5 min puis par un refroidissement lent jusqu'à atteindre la température ambiante. Les oligonucléotides de liaison ont été ajoutés à la concentration finale de 30nM à une quantité de 200ng à 2µg d'ADN de cellules infectées dans un volume final de 20µl en présence de la ligase du *Quick ligation kit* 0.8µl, soit 300 unités d'enzymes (NEB, référence M2200S) pendant 2 heures à température ambiante dans un volume final de 20µL. Les produits ADN ligaturés ont ensuite été purifiés avec le kit *PCR switch charge purification* (Life Technology, CS12000) selon les instructions du fabriquant puis élués dans 20µL et soumis à une PCR en temps réel.

Les quantifications ont été mises en oeuvre par PCR en temps réel sur un appareil *Light Cycler* (Roche Diagnostics, Meylan, France). Les quantifications, par rapport à la gamme utilisée, ont été calculées à l'aide de la méthode de la dérivée seconde fournie par le logiciel de quantification du *Light Cycler,* version 3.5 (Roche Diagnostics).

Dans un premier tour de PCR, 1/10 de l'ADN purifié précédemment a été amplifié dans un mélange réactionnel de 20µL comprenant du tampon 1 x *LightCycler FastStart DNA master Hybprobes* (Roche, référence 12239272001), 4mM MgCl₂, 300nM d'amorces 32t 5'-GCGCGCGGCGGTGACCCGGGAGATCTGAATTC-3' (SEQ ID NO: 25) et MS1 5'-CTCGCCTCTTGCCGTGCGCG-3' (SEQ ID NO : 9).

Afin de rester dans la phase exponentielle, seulement 12 cycles ont été effectués. Ainsi, les conditions du premier tour de PCR étaient les suivantes : une étape de dénaturation de 8 min à 95°C puis 12 cycles d'amplification (95°C pendant 10s, 60°C pendant 10s, et 72°C pendant 31s). Le deuxième tour de PCR emboitée (« *nested PCR* ») a été réalisé sur 1/100 du produit du premier tour de PCR dans un mélange comprenant du tampon 1 x *LightCycler FastStart DNA master Hybprobes*, 4mM MgCl₂, 300nM des amorces 25t (5'-GCGGTGACCCGGGAGATCTGAATTC-3', SEQ ID NO: 5) et MS2 (5'-GAGTCCTGCGTCGAGAGATC-3', SEQ ID NO : 26) et 200nM des sondes d'hybridation MHFL* (5'-CCCTCAGACCCTTTTAGTCAGTGTGGAA^{a}-3', SEQ ID NO : 27) et MHLC* (5'-TCTCTAGCAGTGGCGCCCGAACAG^{b}-3', SEQ ID NO : 28) (a : Fluorescéine en 3' ; b : LCred640 en 5' et phosphorylé en 3').

Le nombre de copies de l'ADN linéaire maturé et non maturé a été déterminé par référence aux courbes de calibration préparées en amplifiant des quantités allant de 10 à 10⁵ copies de fragments digérés correspondant.

*Quantification des cercles 1-LTR.* Les quantifications ont été réalisées par PCR en temps réel sur un appareil *Light Cycler* (Roche Diagnostics, Meylan, France) à l'aide de la méthode de la dérivée seconde fournie par le logiciel de quantification du *Light Cycler,* version 3.5 (Roche Diagnostics). Le nombre de copies d'ADN des cercles 1-LTR a été déterminé en quantifiant les molécules virales à l'aide d'amorces qui s'hybrident aux gènes *gag* et *env,* ainsi que deux sondes d'hybridation. Les mélanges réactionnels contiennent du tampon 1 x *LightCycler FastStart DNA master HybProbes* (Roche Diagnostics référence 12239272001), 4mM MgCl₂, 300nM des amorces 1LTR LA1 (5'-GCGCTTCAGCAAGCCGAGTCCT-3', SEQ ID NO : 1) et 1LTR LA16 (5'-GTCACACCTCAGGTACCTTTAAGACCAATGAC-3', SEQ ID NO : 2) et 200nM des sondes d'hybridation LTR FL* (5'-CACAACAGACGGGCACACACTACTTGA^{a}-3', SEQ ID NO: 3) et LTR LC*(5'-CACTCAAGGCAAGCTTTATTGAGGC^{b}-3', SEQ ID NO : 4) (^{a} Fluorescéine à l'extrémité 3'; ^{b} LC red 640 à l'extrémité 5' et phosphorylé à l'extrémité 3') dans un volume final de 20µl. Les conditions des cycles de PCR pour la quantification des cercles 1-LTR sont les suivantes : étape de dénaturation pendant 8 minutes suivie de 40 cycles (95°C pendant 10s, 60°C pendant 10s, et 72°C pendant 25s). Le nombre de copies des cercles 1-LTR a été déterminé par référence à une courbe de calibration préparée en amplifiant des quantités allant de 100 à 10⁵ copies de p1-LTR.

### RESULTATS

### Quantification de l'ADN linéaire du VIH-1

La quantification de l'ADN viral linéaire a été réalisée en suivant une approche par PCR mettant en oeuvre une ligation (*ligation-mediated PCR,* LM-PCR) apportant des améliorations majeures à celle de Pierson *et al.* (Pierson et al. (2002) Journal of Virology 17 : 8518-8531).

L'efficacité et la sensibilité de la PCR quantitative avec les amorces 25t et MS2 sont représentées pour pLIN-HIV-ScaI (**Figure 1A**). Les paramètres utilisés pour pLIN-HIV-NdeI étaient identiques à ceux déterminés pour pLIN-HIV-ScaI indiquant que la différence des deux plasmides concernant l'extrémité de l'ADN viral (un site *Sca*I pour pLIN-HIV-ScaI et un site *Nde*I pour pLIN-HIV-NdeI) n'influence pas la PCR quantitative, comme attendu.

L'ADN mimant l'extrémité non maturé a été obtenu par digestion par *Sca*I/*Aat*II puis purification. L'ADN mimant l'extrémité 3' maturée a été obtenue par digestion par *Nde*I/*Aat*II puis purification. Après purification, ces fragments ont été quantifiés par PCR quantitative en utilisant les amorces et les sondes pour la quantification de l'ADN viral total (voir le **Tableau 1**). Comme décrit dans la **Figure 1B****,** la LM-PCR avec le linker 11GTb conduit à la détection de l'ADN viral linéaire maturé en 3' et non maturé avec une efficacité de 90% et une sensibilité de 10 copies pour 200 000 cellules dans les deux cas (comme indiqué dans la partie matériels et méthodes l'efficacité a en fait été déterminée à l'aide du linker 11TAb dont le bout collant de 2 nucléotides est complémentaires de celui généré par la coupure *Nde*I mais par souci de simplification c'est le linker 11GTb qui est représenté dans la figure).

La LM-PCR avec l'oligonucléotide de liaison (linker 11b) est seulement capable de détecter et de quantifier l'ADN viral non maturé. En conséquence, la quantité d'ADN maturé en 3' est déduite en soustrayant la quantité obtenue avec l'oligonucléotide de liaison (linker 11GTb) de celle obtenue avec l'oligonucléotide de liaison (linker 11b). Pour chaque quantification (ADN viral linéaire non maturé et ADN linéaire maturé en 3'), plusieurs dilutions des fragments correspondant ont été réalisées dans de l'ADN de cellules non infectées (200ng/µl). Un oligonucléotide de liaison (linker 11GTb pour l'ADN linéaire non maturé et linker 11b pour l'ADN linéaire maturé) a été ligaturé avec chaque dilution d'extrémité d'ADN viral (non maturée ou maturée en 3'). En conséquence, la quantification obtenue après PCR prend en compte l'efficacité de ligation. Après ligation, l'ADN ligaturé a été purifié à l'aide de billes magnétiques selon les instructions du fabricant (Invitrogen) afin d'empêcher l'inhibition de la PCR du fait du mélange de la réaction de ligation. L'ADN ligaturé (1/10 de la réaction de ligation) à alors été soumis à une amplification par PCR en temps réel avec les amorces 32t et MS1 (voir le **Tableau 1**).

Le nombre de cycle a été déterminé de manière empirique. Afin de rester dans la phase exponentielle, seulement 12 cycles de PCR ont été réalisés. De manière inattendue, diminuer le nombre de cycle (par exemple 8 cycles) lors de la première PCR résulte en une amplification faible des échantillons aboutissant à une quantification erronée. De même, augmenter le nombre de cycle n'améliore pas la quantification. Par ailleurs, après 30 cycles, les courbes de la gamme de plasmide utilisées pour la quantification (dilution de 10 en 10) sont quasi superposées; par conséquent, la quantification de l'échantillon est impossible. Ainsi, 12 cycles sont suffisants pour permettre une quantification de toutes les dilutions d'ADN ligaturé à l'issu de la deuxième PCR. Les produits amplifiés (1/100) ont ensuite été soumis à la deuxième PCR. L'efficacité de chaque quantification (non mature et maturée en 3') est décrite dans les **Figure 1B** **et** **1C****.**

Comme indiqué plus haut, il est important de noter que l'extrémité du fragment issu de la digestion par *Nde*I est similaire mais non identique à celui trouvé à l'extrémité 3' dans les cellules infectées (5'-ATTG et non pas 5'-ACTG dans les cellules infectées), ce qui rend nécessaire l'utilisation de l'oligonucléotide de liaison 11TAb pour ces expériences à la place de l'oligonucléotide de liaison 11GTb. Comme décrit par Pierson *et al.* (Pierson et al. (2002) Journal of Virology 17 : 8518-8531), l'oligonucléotide de liaison (linker 11GTb) est capable de détecter l'extrémité virale non maturée. La raison peut être due au fait que le bout collant de l'oligonucléotide de liaison (5'-GT) n'est pas impliqué dans la ligation avec le phosphate du côté 5' du génome. Toutefois, en utilisant cet oligonucléotide de liaison (linker 11GTb), tant l'extrémité non mature que l'extrémité maturée en 3' peuvent être efficacement détectées. Globalement, les inventeurs ont montré que des oligonucléotides de liaison assymétriques peuvent être utilisés pour une quantification efficace des extrémités de l'ADN viral.

Par conséquent, les couples 25t/11GT et 25t/11b seront utilisés pour quantifier à la fois les extrémités non maturées et maturées en 3' pendant le cours naturel de l'infection.

### Quantification des cercles 1-LTR

Plusieurs méthodes basées sur la PCR ont été proposées afin de quantifier les cercles 1-LTR (Bukrinsky et al. (1992) Proc Natl Acad Sci USA 89, 14:6580-4). Ces PCR sont basées sur l'hybridation d'amorces dans les gènes *env* et *gag* (**Figure 2A****1**). Toutefois, il a été rapporté que les méthodes basées sur la PCR ne permettaient pas une quantification correcte des cercles 1-LTR (Yoder & Fishel (2006) Journal of Virological Methods 138,1-2 :201-6). En effet, comme cela est montré dans la **Figure 2A****2**, les amorces utilisées pour les cercles 1-LTR pourraient conduire à une amplification de la région LTR-LTR présente dans les cercles 2-LTR.

De plus, comme décrit par Yoder *et al*., ces amorces pourraient conduire à une amplification linéaire du LTR 5' et du LTR 3' de l'ADN viral linéaire (**Figure 2A****3**). L'ADN simple brin linéaire produit pourrait s'hybrider aux séquences LTR homologue ce qui conduirait à une amplification exponentielle à partir de l'ADN viral et pourrait en conséquence aboutir à des biais dans la quantification des cercles 1-LTR.

L'amplification avec les amorces 1LTR LA1 (5'-GCGCTTCAGCAAGCCGAGTCCT-3', SEQ ID NO: 1) et 1LTR LA16 (5'-GTCACACCTCAGGTACCTTTAAGACCAATGAC-3', SEQID NO : 2) a été réalisée avec de l'ADN de cellules de la lignée cellulaire CEM infectées et le produit d'amplification a été cloné dans le vecteur pGEMT-easy pour donner p1-LTR utilisé comme calibrateur de la PCR en temps réel (**Figure 2B**). Dans ce cadre, il a été déterminé que la durée optimale de l'étape d'élongation de la PCR était de 25 secondes. p2-LTR qui comprend les LTR dans leur intégralité entourant les régions *gag* et *env* est utilisé en tant que témoins (**Figure 2B**). Une PCR quantitative à l'aide des amorces 1LTR LA1 et 1LTR LA16 et avec p1-LTR comme matrice conduit à une détection sensible (**Figures 2C** **et** **2D****, Tableau 2**) et efficace (200 copies/10⁶ cellules) de l'ADN 1-LTR. p2-LTR, soumis au même protocole, conduit à un faible signal d'amplification (**Figures 2C** **et** **2D****, Tableau 2**). De plus, p2-LTR a été digéré par *Sca*I pour mimer l'ADN viral linéaire trouvé dans les cellules infectées. Les inventeurs ont précisément quantifié l'ADN purifié selon le protocole adapté à l'ADN viral total. Des amplifications à partir de plusieurs dilutions de cet ADN dans des cellules non infectées conduit à des signaux d'amplification qui sont négligeables en comparaison avec la quantité initiale d'ADN linéaire (**Figures 2C** **et** **2D****, Tableau 2**), ce qui confirme l'amplification à partir de l'ADN viral linéaire comme montré par Yoder *et al.* (Yoder & Fishel (2006) Journal of Virological Methods 138,1-2 :201-6). L'amplification non spécifique à partir d'ADN linéaire viral a été estimée à moins de 1% par rapport à la quantité initiale. Globalement, l'amplification de p1-LTR avec les amorces et le protocole décrits ici est représentative de la quantification des cercles 1-LTR.

**Tableau 2 : amplifications obtenues à partir du plasmide 1-LTR, du plasmide 2-LTR et de l'ADN mimant l'ADN viral linéaire après digestion du plasmide 2-LTR par l'enzyme ScaI**

| | Quantité initiale | Quantité calculée | Pourcentage d'amplification |
|---|---|---|---|
| p1-LTR | 2.10⁵ | 1,925.10⁵ | 96,2 |
| | 2.10⁴ | 2,109.10⁴ | 100 |
| | 2.10³ | 2,002.10³ | 100 |
| p2-LTR | 2.10⁵ | 1,937.10³ | 0,7 |
| | 2.10⁴ | 2,242.10² | 0,8 |
| | 2.10³ | 5,311.10¹ | 1,9 |
| p2-LTR linéarisé | 2.10⁵ | 6,733.101 | 0,03 |
| | 2.10⁴ | 9,786 | 0,05 |
| | 2.10³ | ND | ND |

### Exemple 2

Afin de valider la méthode décrite dans l'**Exemple 1,** les inventeurs ont quantifié les différentes formes d'ADN viral lors d'une infection de cellules par des virus VIH-1 dérivés de pNL4-3: virus Δenv NLENG1-ES-IRES type sauvage (wt) et NLENG1-ES-IRES D116N (Gelderblom et al. (2008) Retrovirology 9;5:60).

20 millions de cellules CEM sont infectées par un virus pseudotypé par l'enveloppe VSV-G (30ng d'antigène p24 par million de cellule). 3 conditions d'infection sont réalisées : infection par un virus dont l'intégrase est catalytiquement active *(wild type*, WT) en absence ou en présence de 500nM de Raltégravir (RAL), un inhibiteur de la réaction d'intégration (Steigbigel et al. (2008) New England Journal of Medicine 359,4 :339-354). La dernière condition est l'infection par un virus dont l'intégrase est catalytiquement inactive (D116N) (Shin et al. (1994), Journal of Virology 68,3 :1633-1642).

Les résultats attendus sont:
- "WT": "Wild-Type": présence d'ADN maturé due à l'activité de l'intégrase ainsi que la détection d'ADN viral intégré.
- "D116N": Intégrase catalytiquement inactive: absence d'ADN maturé et d'ADN viral intégré due à l'absence d'activité de l'intégrase.
- "RAL": infection par un virus ayant une intégrase "WT" en présence de Raltégravir: présence d'ADN viral maturé car le Raltégravir n'est pas un inhibiteur de la réaction de maturation (*3'-processing*). En revanche, on s'attend à une absence d'ADN viral intégré car le Raltégravir est un inhibiteur de la réaction d'intégration.

1 heure après infection, les cellules sont lavées dans un tampon phosphate (PBS), mises en contact avec la trypsine pendant 1 minute à 37°C. Les cellules sont ensuite lavées une fois dans du milieu de culture et deux fois dans un grand volume de PBS. Les cellules sont ensuite cultivées dans du milieu RPMI 1640 contenant 10% de sérum de veau foetal. A chaque temps post-infection, un échantillon de 3 millions de cellules est constitué. Pour éliminer l'ADN utilisé pour la préparation des virus, le culot cellulaire est soumis à la digestion par 1,500U de DNaseI (Invitrogen, référence AM2238) dans un tampon contenant 20mM Tris-HCl pH 8,3, 50mM KCl, 2mM MgCl₂ pendant 10 minutes à température ambiante. Le culot cellulaire est ensuite lavé dans du PBS et les cellules sont centrifugées puis mises à -80°C avant utilisation. L'ADN est ensuite extrait grâce au kit *QIAamp DNA blood mini kit* (Qiagen, référence 51104).

Le nombre de copies d'ADN viral total est déterminé en utilisant des amorces (Butler et al. (2001) Nature Medicine 7:631-634) s'hybridant dans la région U5 du LTR (amorce MH531 : 5'-TGTGTGCCCGTCTGTTGTGT-3', SEQ ID NO: 29) et dans le gène gag (amorce MH532: GAGTCCTGCGTCGAGAGAGC-3', SEQ ID NO: 30) ainsi que les sondes d'hybridation MH FL* (5'-CCCTCAGACCCTTTTAGTCAGTGTGGAAa-3', SEQ ID NO : 31) et MH LC* (5'-TCTCTAGCAGTGGCGCCCGAACAGb, SEQ ID NO : 32) (a : Fluorescéine à l'extrémité 3'; b : LC red 640 à l'extrémité 5' et phosphorylation à l'extrémité 3') (**Tableau 1**).

Les cercles à 2-LTR sont amplifiés par les amorces encadrant la jonction LTR-LTR (amorces HIVF et HIVR1) (Brussel & Sonigo (2003) Journal of Virology 77:10119-10124). L'ADN viral intégré est quantifié par Alu-PCR (Brussel & Sonigo (2003) Journal of Virology 77:10119-10124). Les séquences U5-gag, les jonctions 2-LTR ainsi que l'amplification spécifique de l'ADN intégré sont amplifiés en double à partir d'1/50 de l'extraction d'ADN cellulaire. Le nombre de copies est normalisé par µg d'ADN en mesurant la quantité de gène β-globine dans 1/50 de l'extrait d'ADN (**Tableau 1**).

Les résultats sont présentés dans les **Figure 3A-3F****.** Comme le montrent ces figures, la synthèse de l'ADN viral total, témoin de la transcription inverse, est maximale 10 heures post-infection. La formation des cercles à 2-LTR est maximale 36 heures post-infection. L'intégration du virus est complète 48 heures post-infection. Ces cinétiques sont conformes à celles décrites dans le cas d'une infection par le VIH-1 (Brussel & Sonigo (2003) Journal of Virology 77:10119-10124). La quantité d'ADN viral linéaire (qui comprend les deux sous-populations : maturé et non maturé) est atteinte 8 heures post-infection. Elle est la même pour les 3 conditions, *i.e.* "WT", "RAL" et "D116N" ce qui est normal car ni la mutation D116N de l'intégrase, ni le Raltégravir, n'influence le taux de conversion d'ARN en ADN linéaire par la transcriptase inverse. L'ADN est ensuite dégradé témoignant de sa dégradation par la voie du protéasome (Butler et al. (2002) Journal of Virology 76:3739-47).

La discrimination entre l'ADN viral maturé et non maturé a été ensuite validée suivant le protocole présenté dans l'**Exemple 1.** Les résultats sont présentés dans les **Figures 4A-4D****.**

Le Raltégravir, inhibiteur d'intégrase, est un inhibiteur de la réaction d'intégration et non de la maturation des extrémités dans le contexte d'une infection (Nguyen et al. (2011) Ann. N. Y. Acad Sci. 1222:83-9). L'efficacité de cette drogue est prouvée dans la **Figure 3D** (inhibition de la détection d'ADN viral intégré, similaire à l'inhibition de l'intégration par un mutant du site catalytique (D116N)). La quantité d'ADN viral maturé avec ou sans Raltégravir est similaire (**Figure 4A-4B**). En revanche, l'ADN viral maturé est quasi indétectable dans le cas d'une infection par le mutant du site catalytique qui a été montré comme étant totalement dépourvue d'activité y compris donc pour la catalyse de l'étape de maturation (**Figure 4C**). De plus, le pourcentage d'ADN viral linéaire maturé décroit en fonction du temps post-infection par rapport à l'ADN non maturé (**Figure 4D**). Ainsi, cette expérience met en évidence une moins grande stabilité de l'ADN viral maturé par rapport à l'ADN viral linéaire immature. L'ADN viral linéaire étant la forme virale la moins stable parmi les différents génomes viraux.

Les Styrylquinolines (SQ) sont, *in vitro,* des inhibiteurs de fixation de l'intégrase sur l'ADN viral (Deprez et al. (2004) Mol. Pharmacol. 65:85-98) et représentent donc une classe différente de celle du Raltégravir puisque les SQ agissent comme des inhibiteurs compétitifs de l'étape de maturation. Bien qu'efficace lors d'une infection virale, l'étape ciblée par ces inhibiteurs n'a jamais pu être clairement identifiée par manque d'outil permettant, *ex vivo*, de caractériser l'étape de maturation de l'ADN viral (Bonnenfant et al. (2004) Journal of Virology 78:5728-36). La méthode selon l'invention a donc permis de démontrer que les styrylquinolines, dont la molécule FZ41 est le prototype, inhibe bien la réaction de maturation de l'ADN viral.

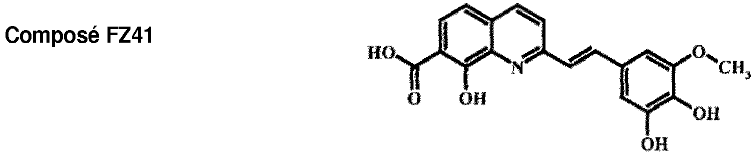

En effet, des cellules CEM ont été infectées par le virus possédant une intégrase catalytiquement active "wild-type". Les cellules ont été traitées ou non, au moment de l'infection, par 20µM de FZ41. 20 heures post-infection l'ADN viral linéaire mature et l'ADN viral immature sont quantifiés et reportés **Figure 5****.** Les résultats montrent une inhibition de la maturation de l'ADN viral par le composé FZ41. En effet, le pourcentage d'ADN viral maturé est de 75% en absence de SQ (et par conséquent 25% d'ADN non maturé) contre 28% d'ADN maturé en présence de FZ41. L'inhibition de la maturation de l'ADN viral est donc importante en présence de SQ.

La méthode selon l'invention, fiable, sensible, peu couteuse, est donc bien adaptée au criblage de composés inhibant l'intégrase en vue de tester leur activité sur la maturation de l'ADN linéaire, seul substrat de la réaction d'intégration.

De plus, cette méthode peut être déclinée sur un test clinique en vue d'un test prédictif d'échappement des patients, la charge virale étant associée à la quantité d'ADN viral dans les cellules infectées.

### Exemple 3

Afin d'évaluer l'influence du contenu en cercles à 2-LTR sur la quantification des cercles à 1-LTR selon l'invention, des cellules Nalm-6 (ligase-4⁺) et Nalm-114 (ligase-4⁻) ont été infectées par des VIH-1 Δenv, soit de type sauvage soit de type D116N (intégrase catalytiquement inactive). Il a précédemment été montré que par marquage de Southern que la ligase-4 est uniquement impliquée dans la formation des cercles à 2-LTR (pas dans la formation de ceux à 1-LTR) et que la présence de la mutation D116N conduit à une forte accumulation de cercles à 2-LTR dans un contexte ligase-4⁺ (et dans une moindre mesure à une légère accumulation de cercles à 1-LTR) du fait d'un défaut d'intégration. Les résultats des inventeurs confirment la forte inhibition (facteur 40) de la formation des cercles à 2-LTR à la fois pour les virus de type sauvage et D116N en cellules Nalm-114 par rapport aux cellules Nalm-6. Les quantités de cercles à 1-LTR sont semblables pour les lignées cellulaires infectées par les virus de type sauvage ou de type D116N. Ces résultats confirment que la ligase-4 n'est pas impliquée dans la formation des cercles à 1-LTR. De façon importante, les inventeurs ont remarqué que la quantité de cercles à 1-LTR était essentiellement inchangée quelle que soit le niveau d'accumulation des cercles à 2-LTR, ce qui confirme qu'à l'aide de l'approche quantitative de l'invention, une quantification exacte des cercles à 1-LTR dans le contexte cellulaire et possible sans biais lié à la présence de cercles à 2-LTR.

### Exemple 4

Deux autres inhibiteurs d'intégrase (INSTI), l'élvitégravir (EVG) et le dolutégravir (DTG), ont été étudié pour leur capacité à inhiber la réaction de maturation (*3'-processing*) et ont été systématiquement comparés au raltégravir (RAL). Aux plus faibles concentrations de RAL, DTG et EVG inhibant complètement l'intégration virale (500 nM) (les trois inhibiteurs présentent des IC₅₀ similaires en ce qui concerne l'inhibition de l'intégration virale, 2 nM pour DTG et EVG, 8 nM pour RAL), l'étape de maturation n'a pas été affectée quelle que soit l'inhibiteur utilisé. De manière intéressante, l'augmentation de la concentration en inhibiteur a eu des effets différents sur cette réaction de maturation selon le composé. Ainsi, RAL est resté essentiellement inefficace jusqu'à des concentrations de 5 µM où 80% de l'activité de maturation était encore visible. A *contrario*, l'activité de maturation était grandement affectée à 2,5 ou 5 µM de DTG et d'EVG (efficacité de maturation à environ 40% et inférieure à 25% à 2,5 µM et 5 µM respectivement).

De fait, les études *in vitro* à l'aide d'intégrase recombinante montrent que les trois inhibiteurs n'inhibent pas la réaction de maturation au même niveau. Les IC₅₀ relative à la réaction de maturation sont respectivement de 2 µM, 5 µM et 10 µM pour DTG, EVG et RAL.

Les effets différents de chacun des inhibiteurs sur la réaction de maturation ont été confirmés à l'aide d'une sonde radiomarquée dérivé de la PCR. 500 nM de chaque inhibiteur ont conduits à des quantités similaires d'ADN maturé, alors qu'à 5 µM d'inhibiteur, DTG et EVG inhibent significativement la maturation, au contraire de RAL.

De manière intéressante, varier les concentrations de DTG et d'EVG, de 500 nM à 5 µM, augmente de manière continue l'inhibition de la maturation mais ne conduit pas à une augmentation de l'accumulation de cercles à 2-LTR ou à 1-LTR. En effet, à cette gamme de concentrations où DTG et EVG inhibent complètement l'intégration, les cercles à 2 LTR représentent quasiment 40% des formes virales à 48 h post-infection. Ces données démontrent que l'accumulation de cercles à 2-LTR est principalement reliée à l'inhibition de l'intégration et non à l'efficacité de la réaction de maturation.

L'efficacité de maturation a été mesurée pendant l'infection de lymphocytes T CD4+ primaires à l'aide d'un virus Δenv de type sauvage précédemment décrit (+/-RAL, EVG ou DTG à 5 µM). On observe que l'efficacité de la réaction de maturation est élevée (75%) mais légèrement ralentie dans les cellules primaires (le maximum de maturation est atteint 24 h post-infection) et influencée par EVG et DTG (à 5 µM) mais pas par RAL. De plus les cinétiques d'accumulation des cercles à 1-LTR et à 2-LTR sont similaires dans les cellules primaires par rapport aux cellules de lignée cellulaire.

### Exemple 5

A l'heure actuelle, l'origine de la formation de cercles à 1-LTR reste peu claire. Ainsi, pour certains auteurs, la formation des cercles à 1-LTR requiert une recombinaison homologue entre 2 LTR d'ADN linéaire dans le noyau alors que pour d'autres elle implique une étape de transcription inverse dans le compartiment cytoplasmique.

Un fractionnement cellulaire a été effectué 24 h post-infection sur des cellules MT4 infectées avec un virus Δenv porteur de la mutation D116N décrit précédemment, c'est-à-dire lorsque l'import nucléaire est accompli, comme en témoigne la quantité maximale de cercles à 2-LTR qui sont formés exclusivement dans le noyau. En effet, les résultats des inventeurs confirment que plus de 99,5% des cercles à 2-LTR sont retrouvés dans le noyau. En comparaison, la quantité de cercles à 1-LTR était beaucoup plus importante dans la fraction cytoplasmique. En effet, dans des conditions ou l'import nucléaire est maximal, les cercles à 1-LTR formés dans le cytoplasme représentent 10% de la quantité totale de cercle à 1-LTR. Ceci est cohérent avec la cinétique de formation des cercles à 1-LTR où les cercles à 1-LTR représentent 10% de l'ADN viral dès 5 h et 8 h post-infection et compatible avec l'hypothèse selon laquelle au moins une partie des cercles à 1-LTR pourraient être formés pendant l'étape de transcription inverse.

Les peptides NLS-IN-Pen et SV40-NLS-Pen, qui ont été décrit comme inhibant l'import nucléaire du complexe de pré-intégration via l'inhibition de l'interaction entre l'intégrase et l'importine α (Levin et al. (2009) Retrovirology 6:112), ont été utilisés pour déterminer si les cercles à 1-LTR sont formés uniquement lors de l'étape de transcription inverse et ne nécessite pas la translocation du complexe de pré-intégration. Des cellules HeLa, traitées par l'un des deux peptides, ont été infectées avec un virus pNL4.3 en présence de RAL. Sans peptide, les cercles à 2-LTR s'accumulent jusqu'à représenter 19,3% de l'ADN viral total à 48h post-infection. Le traitement par les peptides conduit à une inhibition de l'accumulation de cercles à 2-LTR (5,22% et 2,24 pour NLS-IN-Pen et SV40-NLS-Pen respectivement) soulignant l'inhibition de l'import nucléaire (3,7 et 8,6 fois pour NLS-IN-Pen et SV40-NLS-Pen respectivement). De manière intéressante, dans ces conditions, les inventeurs observent une diminution de la formation des cercles à 1-LTR mais moins marquée que pour l'inhibition relative aux cercles à 2-LTR (1,8 et 2,5 fois pour NLS-IN-Pen et SV40-NLS-Pen respectivement).

L'inhibition de l'import nucléaire du complexe de pré-intégration a pu également être effectuée plus spécifiquement par des mutations dans les régions FLAP et/ou CTS du virus. Ainsi, des cellules HeLa ont été infectées par un mutant défectif, à la fois dans les régions CTS et PPT. Les quantités de cercles à 1-LTR et à 2-LTR ont été normalisées par rapport aux quantités déterminées en conditions de type sauvage à 24 h post-infection. Bien que les inventeurs ont trouvé que l'abrogation de la structure FLAP inhibe partiellement, et non complètement, l'import nucléaire du complexe de pré-intégration, les inventeurs ont observé une diminution substantielle de la quantité de cercles à 2-LTR (environ 2 fois). Dans ce contexte, les inventeurs ont mis en évidence une diminution concomitante de la quantité de cercles à 1-LTR mais à un degré moindre (1,4 fois). Prises ensemble, ces données suggèrent clairement que les deux mécanismes de formation des cercles à 1-LTR ne sont pas mutuellement exclusifs : des cercles à 1-LTR pourraient être formés dans le cytoplasme lors de la phase de transcription inverse mais la majeure partie des cercles à 1-LTR (90%) serait formée par recombinaison homologue, dans le noyau, après la translocation du complexe de pré-intégration.

**Tableau 1**

| Nom de la sonde ou de l'amorce | Séquence | SEQ ID NO : | Cible | Dénaturation | Cycles PCR |
|---|---|---|---|---|---|
| MH 531 | 5'-TGTGTGCCCGTCTGTTGTGT | 29 | ADN total du VIH-1 | 95°C, 8min | 95°C 10s, 60°C 10s, 72°C 6s, 50 cycles |
| MH 532 | 5'-GAGTCCTGCGTCGAGAGAGC | 30 | | | |
| | | | | | |
| MH FL* | 5'-CCCTCAGACCCTTTTAGTCAGTGTGGAA^{a} | 31 | | | |
| MH LC* | 5'-TCTCTAGCAGTGGCGCCCGAACAG^{b} | 32 | | | |
| HIV F | 5'-GTGCCCGTCTGTTGTGTGACT | 33 | Cercles 2-LTR | 95°C, 8min | 95°C 10s, 66°C 10s, 72°C 10s, 15 cycles puis 55 cycles avec une baisse de la température d'hybridation de 0.5°C/cycle jusqu'à 59°C |
| HIV R1 | 5'-ACTGGTACTAGCTTGTAGCACCATCCA | 34 | | | |
| | | | | | |
| HIV FL* | 5'-CCACACACAAGGCTACTTCCCTGA^{a} | 35 | | | |
| HIV LC* | 5'-TGGCAGAACTACACACCAGGGC^{b} | 36 | | | |
| L-M667 | 5'-ATGCCACGTAAGCGAAACTCTGGCTAACTAGGGAACCCACTG | 37 | ADN intégré du VIH-1 | 95°C, 8min | 95°C 10s, 60°C 10s, 72°C 170s, 12 cycles |
| Alu1 | 5'-TCCCAGCTACTGGGGAGGCTGAGG | 38 | (premier tour de PCR) | | |
| Alu2 | 5'-GCCTCCCAAAGTGCTGGCATTACAG | 39 | | | |
| | | | | | |
| Lambda | 5'-TATGCCACGTAAGCGAAACT | 40 | ADN intégré du VIH-1 | 95°C, 8min | 95°C 10s, 60°C 10s, 72°C 9s, 50 cycles |
| AA55M | 5'-GCTAGAGATTTTCCACACTGACTAA | 41 | (deuxième tour de PCR) | | |
| | | | | | |
| LTR FL* | 5'-CACAACAGACGGGCACACACTACTFGA^{a} | 3 | | | |
| LTR LC* | 5'-CACTCAAGGCAAGCTTTATTGAGGC^{b} | 4 | | | |
| 32t | 5'-GCGCGCGGCGGTGACCCGGGAGATCTGAATTC | 25 | ADN linéaire du VIH-1 | 95°C, 8min | 95°C 10s, 60°C 10s, 72°C 31s, 12 cycles |
| MS1 | 5'-CTCGCCTCTTGCCGTGCGCG | 9 | (premier tour de PCR) | | |
| | | | | | |
| 25t | 5'-GCGGTGACCCGGGAGATCTGAATTC | 5 | ADN linéaire du VIH-1 | 95°C, 8min | 95°C 10s, 60°C 10s, 72°C 26s, 50 cycles |
| MS2 | 5'-GAGTCCTGCGTCGAGAGATC | 26 | (2^{ème} tour de PCR) | | |
| | | | | | |
| MH FL* | 5'-CCCTCAGACCCTTTTAGTCAGTGTGGAA^{a} | 27 | | | |
| MH LC* | 5'-TCTCTAGCAGTGGCGCCCGAACAG^{b} | 28 | | | |
| 1LTR LA1 | 5'-GCGCTTCAGCAAGCCGAGTCCT | 1 | Cercles 1-LTR | 95°C, 8min | 95°C 10s, 60°C 10s, 72°C 25s, for 50 cycles |
| 1LTR LA16 | 5'-GTCACACCTCAGGTACCTTTAAGACCAATGAC | 2 | | | |
| | | | | | |
| LTR FL* | 5'-CACAACAGACGGGCACACACTACTTGA^{a} | 3 | | | |
| LTR LC* | 5'-CACTCAAGGCAAGCTTTATTGAGGC^{b} | 4 | | | |

| | | | | | |
|---|---|---|---|---|---|
| a Modifié par de la fluorescéine en 3'. b Modifié par le fluorophore LC red 640 en 5' phosphorylé en 3'. Les amorces et les sondes ont été obtenues de TIB MOLBIOL (Berlin, Allemagne). * sonde | | | | | |

## Revendications

1. Procédé de quantification de la forme linéaire maturée en 3' du génome ADN du virus de l'immunodéficience humaine 1 (VIH-1) dans un échantillon, dans lequel :
(i) on quantifie les formes linéaires totales du génome du VIH-1, où la quantification de l'étape (i) est réalisée par PCR quantitative à partir du produit de ligation obtenu en effectuant une réaction de ligation avec un oligonucléotide double brin qui permet une ligation avec la forme linéaire maturée en 3' et la forme linéaire non maturée en 3' du génome ADN du VIH-1 et comprend un bout collant de deux nucléotides de séquence 5'-GT-3' et
(ii) on quantifie la forme linéaire non maturée du génome ADN du VIH-1 dans lequel on déduit la quantité de la forme linéaire maturée en 3' en soustrayant la quantité de la forme linéaire non maturée du génome ADN du VIH-1 quantifiée à l'étape (ii) de la quantité totale des formes linéaires du génome du VIH-1 quantifiée à l'étape (i) où l'étape (ii) est réalisée par PCR quantitative à partir du produit de ligation obtenu en effectuant une réaction de ligation avec un oligonucléotide double brin qui permet une ligation spécifiquement avec la forme linéaire non maturée en 3' du génome ADN du VIH-1 et comprend un bout franc.

2. Procédé selon la revendication 1, dans lequel la PCR quantitative est réalisée en deux étapes : une première étape d'amplification sans quantification de l'acide nucléique amplifié et une deuxième étape d'amplification avec quantification de l'acide nucléique amplifié.

3. Procédé selon la revendication 2, dans lequel la première étape de la PCR quantitative comprend de 9 à 30 cycles.

4. Procédé selon la revendication 2 ou 3, dans lequel la première étape de la PCR quantitative comprend de 10 à 14 cycles.

5. Procédé selon l'une des revendications 1 à 4, dans lequel la PCR quantitative est une PCR en temps réel.

6. Procédé selon la revendication 5, dans lequel la PCR en temps réel est conduite avec des sondes d'hybridation.

7. Procédé de criblage de composés susceptibles de modifier la quantité d'une forme non intégrée du génome du VIH-1, comprenant la mise en oeuvre d'un procédé tel que défini dans l'une des revendications 1 à 6.

8. Procédé de criblage de composés selon la revendication 7, dans lequel les composés sont susceptibles d'inhiber la maturation de la forme linéaire du génome ADN du VIH-1 et on met en oeuvre un procédé tel que défini dans l'une des revendications 1 à 6.

## Patentansprüche

1. Quantifizierungsverfahren der gereiften linearen Form im 3' des DNA-Genoms des menschlichen Immunschwächevirus 1 (HIV-1) in einer Probe, in der:
(i) totale lineare Formen des Genoms des HIV-1 quantifiziert werden, wobei die Quantifikation des Schritts (i) per quantitativer PCR ausgehend von dem Ligationsprodukt realisiert ist, das per Durchführung einer Ligationsreaktion mit einem doppelsträngigen Oligonukleid erhalten ist, das eine Ligation mit der gereiften linearen Form in 3' und der nicht gereiften linearen Form in 3' des DNA-Genoms des HIV-1 zulässt und ein kohäsives Ende von zwei Nukleotiden der Sequenz 5' - GT-3' umfasst und
(ii) die nicht gereifte lineare Form des DNA-Genoms des HIV-1 quantifiziert wird
bei dem die Menge der gereiften linearen Form in 3' durch Substraktion der Menge der nicht gereiften linearen Form des DNA-Genoms des HIV-1 abgezogen wird, die in Schritt (ii) der Gesamtmenge der linearen Formen des Genoms des HIV-1 quantifiziert ist, die in Schritt (i) quantifiziert ist, in der der Schritt (ii) per quantitativer PCR ausgehend von dem Ligationsprodukt realisiert ist, das per Durchführen einer Ligationsreaktion mit einem doppelsträngigen Oligonukleotid erhalten ist, das eine Ligation spezifisch mit der nicht gereiften linearen Form in 3' des DNA-Genoms des HIV-1 zulässt und ein freies Ende umfasst.

2. Verfahren gemäß Anspruch 1, bei dem die quantitative PCR in zwei Schritten realisiert ist: einem ersten Amplifizierungsschritt ohne Quantifizierung der amplifizierten Nukleinsäure und einem zweiten Amplifizierungsschritt mit Quantifizierung der amplifizierten Nukleinsäure.

3. Verfahren gemäß Anspruch 2, bei dem der erste Schritt der quantitativen PCR 9 bis 30 Zyklen umfasst.

4. Verfahren gemäß Anspruch 2 oder 3, bei dem der erste Schritt der quantitativen PCR 10 bis 14 Zyklen umfasst.

5. Verfahren gemäß einem der Ansprüche 1 bis 4, bei dem die quantitative PCR eine PCR in Echtzeit ist.

6. Verfahren gemäß Anspruch 5, bei dem die PCR in Echtzeit mit Hybridationssonden geführt ist.

7. Aussiebungsverfahren von Verbindungen, die geeignet sind, die Menge einer nicht integrierten Form des Genoms des HIV-1 zu modifizieren, umfassend die Umsetzung eines Verfahrens gemäß Definition in einem der Ansprüche 1 bis 6.

8. Aussiebungsverfahren von Verbindungen gemäß Anspruch 7, bei dem die Verbindungen geeignet sind, die Reifung der linearen Form des DNA-Genoms des HIV-1 zu inhibieren und ein Verfahren gemäß Definition in einem der Ansprüche 1 bis 6 umgesetzt wird.

## Claims

1. A method for quantising the 3' maturated linear form of the DNA genome of the human immunodeficiency virus (HIV-1) in a sample, wherein:
(i) the total linear forms of the HIV-1 genome are quantised, where the quantisation of step (i) is made by quantitative PCR from the ligation product obtained by performing a ligation reaction with a double strained oligonucleotide which allows a ligation with the 3' maturated linear form and the 3' non-maturated linear form of the DNA genome of HIV-1 and comprises a cohesive end of two nucleotides of the sequence 5'-GT-3' and
(ii) the non-maturated linear form of the DNA genome of HIV-1 is quantised
wherein the amount of 3' maturated linear form is deducted by subtracting the amount of non-maturated linear form of the DNA genome of HIV-1 quantised in step (ii) from the total amount of the linear forms of the HIV-1 genome quantised in step (i) where step (ii) is made by quantitative PCR from the ligation product obtained by performing a ligation reaction with a double strain oligonucleotide which allows a specific ligation with the 3' non-maturated linear form of the DNA genome of HIV-1 and comprises a blunt end.

2. The method according to claim 1, wherein the quantitative PCR is made in two steps: a first amplification step without quantising the amplified nucleic acid and a second amplification step including quantising of the amplified nucleic acid.

3. The method according to claim 2, wherein the first step of the quantitative PCR comprises from 9 to 30 cycles.

4. The method according to claim 2 or 3, wherein the first step of the quantitative PCR comprises from 10 to 14 cycles.

5. The method according to one of claims 1 to 4, wherein the quantitative PCR is a real time PCR.

6. The method according to claim 5, wherein the real time PCR is conducted with hybridisation probes.

7. A method for screening compounds likely to modify the amount of a non-integrated form of the HIV-1 genome, comprising the implementation of a method as defined in one of claims 1 to 6.

8. The method for screening compounds according to claim 7, wherein the compounds are likely to inhibit the maturation of the linear form of the DNA genome of HIV-1 and a method as defined in one of claims 1 to 6 is implemented.


### LISTAGE DES SEQUENCES

<110> Centre National de la Recherche Scientifique
   Ecole Normale Supérieure de Cachan
   Université Paris Diderot - Paris 7
<120> METHODE DE QUANTIFICATION DES DIFFERENTES FORMES VIRALES DE L'ADN DU VIH
<130> F644-343
<160> 41
<170> PatentIn version 3.5
<210> 1
   <211> 22
   <212> ADN
   <213> Séquence artificielle
<220>
   <223> Amorce PCR
<400> 1
   gcgcttcagc aagccgagtc ct 22
<210> 2
   <211> 32
   <212> ADN
   <213> Séquence artificielle
<220>
   <223> Amorce PCR
<400> 2
   gtcacacctc aggtaccttt aagaccaatg ac 32
<210> 3
   <211> 27
   <212> ADN
   <213> Séquence artificielle
<220>
   <223> Sonde d'hybridation
<400> 3
   cacaacagac gggcacacac tacttga 27
<210> 4
   <211> 25
   <212> ADN
   <213> Séquence artificielle
<220>
   <223> Sonde d'hybridation
<400> 4
   cactcaaggc aagctttatt gaggc 25
<210> 5
   <211> 25
   <212> ADN
   <213> Séquence artificielle
<220>
   <223> Oligonucléotide de liaison
<400> 5
   gcggtgaccc gggagatctg aattc 25
<210> 6
   <211> 13
   <212> ADN
   <213> Séquence artificielle
<220>
   <223> Oligonucléotide de liaison
<400> 6
   gtgaattcag atc 13
<210> 7
   <211> 11
   <212> ADN
   <213> Séquence artificielle
<220>
   <223> Oligonucléotide de liaison
<400> 7
   gaattcagat c 11
<210> 8
   <211> 51
   <212> ADN
   <213> Séquence artificielle
<220>
   <223> Amorce PCR
<400> 8
   gcggtgaccc gggagatctg aattcagtac tggaagggct aatttggtcc c 51
<210> 9
   <211> 20
   <212> ADN
   <213> Séquence artificielle
<220>
   <223> Amorce PCR
<400> 9
   ctcgcctctt gccgtgcgcg 20
<210> 10
   <211> 6
   <212> ADN
   <213> Séquence artificielle
<220>
   <223> Site de restriction
<400> 10
   agtact 6
<210> 11
   <211> 6
   <212> ADN
   <213> Séquence artificielle
<220>
   <223> Site de restriction muté
<400> 11
   agaact 6
<210> 12
   <211> 43
   <212> ADN
   <213> Séquence artificielle
<220>
   <223> Amorce PCR
<400> 12
   cccgagagct gcatccggag aactacaaag actgctgaca tcg 43
<210> 13
   <211> 43
   <212> ADN
   <213> Séquence artificielle
<220>
   <223> 14
<400> 13
   cgatgtcagc agtctttgta gttctccgga tgcagctctc ggg 43
<210> 14
   <211> 6
   <212> ADN
   <213> Séquence artificielle
<220>
   <223> Site de restriction
<400> 14
   catatg 6
<210> 15
   <211> 44
   <212> ADN
   <213> Séquence artificielle
<220>
   <223> Amorce PCR
<400> 15
   ccgggagatc tgaattcagt catatggaag ggctaatttg gtcc 44
<210> 16
   <211> 44
   <212> ADN
   <213> Séquence artificielle
<220>
   <223> Amorce PCR
<400> 16
   ggaccaaatt agcccttcca tatgactgaa ttcagatctc ccgg 44
<210> 17
   <211> 50
   <212> ADN
   <213> Séquence artificielle
<220>
   <223> Amorce PCR
<400> 17
   cgggagatct gaattcagta ctgctagaga ttttccacac tgactaaaag 50
<210> 18
   <211> 6
   <212> ADN
   <213> Séquence artificielle
<220>
   <223> Site de restriction muté
<400> 18
   agcact 6
<210> 19
   <211> 30
   <212> ADN
   <213> Séquence artificielle
<220>
   <223> Amorce PCR
<400> 19
   agctgcatcc ggagcacttc aagaactgct 30
<210> 20
   <211> 30
   <212> ADN
   <213> Séquence artificielle
<220>
   <223> Amorce PCR
<400> 20
   agcagttctt gaagtactcc ggatgcagct 30
<210> 21
   <211> 6
   <212> ADN
   <213> Séquence artificielle
<220>
   <223> Site de restriction muté
<400> 21
   aatact 6
<210> 22
   <211> 34
   <212> ADN
   <213> Séquence artificielle
<220>
   <223> Amorce PCR
<400> 22
   gtgactggtg aatactcaac caagtcattc tgag 34
<210> 23
   <211> 34
   <212> ADN
   <213> Séquence artificielle
<220>
   <223> Amorce PCR
<400> 23
   ctcagaatga cttggttgag tattcaccag tcac 34
<210> 24
   <211> 13
   <212> ADN
   <213> Séquence artificielle
<220>
   <223> Oligonucléotide de liaison
<400> 24
   tagaattcag atc 13
<210> 25
   <211> 32
   <212> ADN
   <213> Séquence artificielle
<220>
   <223> Amorce PCR
<400> 25
   gcgcgcggcg gtgacccggg agatctgaat tc 32
<210> 26
   <211> 20
   <212> ADN
   <213> Séquence artificielle
<220>
   <223> 27
<400> 26
   gagtcctgcg tcgagagatc 20
<210> 27
   <211> 28
   <212> ADN
   <213> Séquence artificielle
<220>
   <223> Sonde d'hybridation
<400> 27
   ccctcagacc cttttagtca gtgtggaa 28
<210> 28
   <211> 24
   <212> ADN
   <213> Séquence artificielle
<220>
   <223> Sonde d'hybridation
<400> 28
   tctctagcag tggcgcccga acag 24
<210> 29
   <211> 20
   <212> ADN
   <213> Séquence artificielle
<220>
   <223> Amorce PCR
<400> 29
   tgtgtgcccg tctgttgtgt 20
<210> 30
   <211> 20
   <212> ADN
   <213> Séquence artificielle
<220>
   <223> Amorce PCR
<400> 30
   gagtcctgcg tcgagagagc 20
<210> 31
   <211> 28
   <212> ADN
   <213> Séquence artificielle
<220>
   <223> Sonde d'hybridation
<400> 31
   ccctcagacc cttttagtca gtgtggaa 28
<210> 32
   <211> 24
   <212> ADN
   <213> Séquence artificielle
<220>
   <223> Sonde d'hybridation
<400> 32
   tctctagcag tggcgcccga acag 24
<210> 33
   <211> 21
   <212> ADN
   <213> Séquence artificielle
<220>
   <223> Amorce PCR
<400> 33
   gtgcccgtct gttgtgtgac t 21
<210> 34
   <211> 27
   <212> ADN
   <213> Séquence artificielle
<220>
   <223> Amorce PCR
<400> 34
   actggtacta gcttgtagca ccatcca 27
<210> 35
   <211> 24
   <212> ADN
   <213> Séquence artificielle
<220>
   <223> Sonde d'hybridation
<400> 35
   ccacacacaa ggctacttcc ctga 24
<210> 36
   <211> 22
   <212> ADN
   <213> Séquence artificielle
<220>
   <223> Sonde d'hybridation
<400> 36
   tggcagaact acacaccagg gc 22
<210> 37
   <211> 42
   <212> ADN
   <213> Séquence artificielle
<220>
   <223> Amorce PCR
<400> 37
   atgccacgta agcgaaactc tggctaacta gggaacccac tg 42
<210> 38
   <211> 24
   <212> ADN
   <213> Séquence artificielle
<220>
   <223> Amorce PCR
<400> 38
   tcccagctac tggggaggct gagg 24
<210> 39
   <211> 25
   <212> ADN
   <213> Séquence artificielle
<220>
   <223> Amorce PCR
<400> 39
   gcctcccaaa gtgctgggat tacag 25
<210> 40
   <211> 20
   <212> ADN
   <213> Séquence artificielle
<220>
   <223> Amorce PCR
<400> 40
   tatgccacgt aagcgaaact 20
<210> 41
   <211> 25
   <212> ADN
   <213> Séquence artificielle
<220>
   <223> Amorce PCR
<400> 41
   gctagagatt ttccacactg actaa 25